# FASCICULE DE BREVET EUROPEEN

(11) **EP 0 686 391 B1**
(45) Date de publication et mention de la délivrance du brevet: **08.03.2000**
(21) Numéro de dépôt: 95401082.3
(22) Date de dépôt: 10.05.1995
(51) Int. Cl.: A61K 7/48, A61K 7/00, A61K 47/02, B01F 17/54

(54) **Emulsion eau-dans-huile sans tensioactif**
Tensidefreie Wasser-in-Öl Emulsion
Surfactant-free water-in-oil emulsion

(30) Priorité: 06.06.1994 FR 9406899
(43) Date de publication de la demande: 13.12.1995
(73) Titulaire: L'OREAL, 75008 Paris (FR)
(72) Inventeur: Collin, Nathalie, F-92330 Sceaux (FR); Candau, Didier, F-91570 Bievres (FR)
(74) Mandataire: Lhoste, Catherine

(56) Documents cités:
- FR-A- 2 208 642
- COLLOIDS AND SURFACES, vol.38, 1989, AMSTERDAM (NL) pages 325 - 343 S. LEVINE 'Stabilization of Emulsions by Fine Particles I. Partitioning of Particles Between Continous Phase and Oil/Water Interface'
- PATENT ABSTRACTS OF JAPAN vol. 14, no. 30 (C-678) (3973) 19 Janvier 1990 & JP-A-01 268 615 (KAO CORPORATION) 26 Octobre 1989
- J. DISPERSION SCIENCE AND TECHNOLOGY, vol.11, no.1, 1990, USA pages 69 - 74 S. MAGDASSI 'Chitin Particles as O/W Stabilizers.'

## Description

La présente invention se rapporte à une nouvelle émulsion eau-dans-huile (E/H) ne comportant pas de tensioactif. Cette émulsion se présente sous forme d'une crème blanche ou colorée, destinée notamment au traitement cosmétique de la peau (corps, visage). Cette crème peut, en outre, servir au traitement dermatologique de la peau.

Dans le domaine cosmétique, il est courant d'utiliser des crèmes constituées d'une émulsion eau-dans-huile, car elles permettent de former à la surface de la peau un film qui prévient la perte d'eau transépidermique et protège la peau des agressions extérieures. Ces émulsions comportent une phase aqueuse dispersée dans une phase huileuse et un tensioactif qui stabilise la dispersion obtenue. Elles présentent l'inconvénient de contenir des tensioactifs ; or, on sait que les tensioactifs peuvent se montrer irritants vis à vis de certains types de peau.

De plus, de façon classique, ces émulsions contiennent de 25 % à 30 % en poids d'huile, par rapport au poids total de l'émulsion, et ce pourcentage se révèle souvent insuffisant quand on souhaite incorporer une grande quantité d'actifs lipophiles. En outre, quand l'émulsion contient une proportion assez importante d'huile, elle paraît très grasse à l'utilisateur, d'autant que la phase externe de l'émulsion est la phase grasse.

Il subsiste donc le besoin d'une émulsion eau-dans-huile ne présentant pas les inconvénients rencontrés avec celles connues jusqu'à présent, notamment ne contenant pas de tensioactif et comportant une quantité importante d'huile sans avoir la sensation de gras à l'application.

L'émulsion selon l'invention permet justement de remédier aux inconvénients décrits ci-dessous. En effet, la demanderesse a trouvé de manière inattendue qu'il était possible d'obtenir une émulsion eau-dans-huile sans tensioactif en utilisant des particules sphériques siliconées qui stabilisent l'émulsion. Celle-ci a une texture agréable, un toucher poudré et très doux, et un effet frais malgré une quantité importante d'huile.

La présente invention a donc pour objet une émulsion d'une phase aqueuse dans une phase huileuse, contenant des particules sphériques solides finement divisées de polyalkylsilsesquioxane, caractérisée en ce qu'elle est exempte de tensioactif, les particules assurant la dispersion de la phase aqueuse dans la phase huileuse, et en ce qu'elle présente des globules ayant un diamètre de 100 nm à 20 µm. Elle contient, en outre, avantageusement un milieu cosmétiquement et/ou pharmaceutiquement acceptable.

L'invention a aussi pour objet une utilisation de l'émulsion définie ci-dessus pour le traitement cosmétique de la peau (nutrition, hydratation, protection) ainsi que pour la préparation d'une crème destinée au traitement des maladies de la peau (peau sèche).

L'invention a encore pour objet un procédé de traitement cosmétique de la peau consistant à appliquer l'émulsion définie ci-dessus sur la peau.

Certes, il est connu par l'article de C. Levine (Colloids and surfaces, 1989, volume 38, pages 325 à 343, "Stabilization of emulsions by fine particles. I. Partitioning of particles between continuous phase and oil / water interface") d'utiliser des particules fines de silice, rendues hydrophobes par traitement de surface, pour stabiliser des dispersions ne contenant pas de tensioactif, mais ce document ne permet pas de conduire l'homme du métier du domaine cosmétique à l'invention, d'une part parce qu'il concerne un domaine technique tout à fait différent, celui des huiles lourdes et des bitumes, et, d'autre part, parce que l'enseignement qui y est donné conduit à un système liquide à trois phases et non à un système homogène. De plus, les dispersions obtenues sont des dispersions grossières dont les gouttelettes sont visibles à l'oeil nu. Or, par définition, une émulsion cosmétique est une dispersion fine et homogène de deux phases l'une dans l'autre, dont, en pratique les globules ont un diamètre de 100 nm à 20 µm.

Il n'y avait donc pas de raison pour l'homme du métier d'utiliser de telles particules pour stabiliser une émulsion cosmétique, d'autant qu'il est bien connu qu'une émulsion casse très facilement dès qu'on en modifie les constituants et/ou les proportions.

Il est par ailleurs connu selon le document FR-A-2208642 une composition cosmétique liquide à deux phases distinctes, l'une étant constituée d'eau et de solvant organique (éthanol ou propanol) et l'autre étant constituée d'huile, des particules finement divisées étant situées à l'interface des deux phases. Les particules sont constituées de substances minérales ou de substances organiques synthétiques telles que le chlorure de polyvinyle. Ces particules sont destinées à faciliter la formation des billes d'huile dans le milieu hydroalcoolique.

Du fait du type de produit obtenu, à savoir une composition à deux phases, et du fait de la présence d'alcool, ce document ne pouvait pas conduire l'homme du métier à utiliser des particules siliconées pour stabiliser une émulsion eau-dans-huile ne comportant pas d'alcool. Dans la présente invention, on cherche à obtenir une émulsion homogène, la moins irritante possible, donc sans alcool, et non une composition biphasique contenant de l'alcool ou des tensioactifs qui peuvent se révéler irritants.

En outre, les particules décrites dans le document FR-A-2208642 sont, de par leur nature et de par leur taille, différentes de celles qui permettent d'émulsionner l'eau dans l'huile selon l'invention.

Aussi, l'invention a encore pour objet l'utilisation de particules de polyalkylsilsesquioxane pour disperser une phase aqueuse dans une phase huileuse et stabiliser l'émulsion obtenue exempte de tensioactif, la dite émulsion présentant des globules dont le diamètre va de 100 nm à 20 µm.

Les particules selon l'invention sont obtenues notamment soit par traitement de surface de silices pyrogénées, soit par polymérisation d'un alcoxyalkylsilane. Ces procédés conduisent à des particules répondant à la formule A donnée ultérieurement.

Le traitement de surface des silices pyrogénées peut être réalisé à l'aide d'un alkylsilane, éventuellement halogéné, dont le radical alkyle comprend de 1 à 4 atomes de carbone. Le radical alkyle est de préférence un groupe méthyle. Le traitement de surface est, par exemple, réalisé avec un méthylsilane ou un méthylsilane halogéné, et en particulier Me₃SiCl ou ME₂SiCl₂ ou encore MeSiCl₃. Comme particules de ce type utilisables dans l'invention, on peut citer celles vendues sous les dénominations d'Aerosil R805, Aerosil R812 et Aerosil R974 par la société Degussa.

La polymérisation d'alcoxyalkylsilane peut être réalisée à partir d'un alcoxyalkylsilane dont le groupe alcoxy comprend de 1 à 5 atomes de carbone et dont le groupe alkyle comprend de 1 à 4 atomes de carbone. Il s'agit de préférence d'un trialcoxyméthylsilane, et mieux encore du triméthoxyméthylsilane. Un tel procédé de polymérisation est décrit dans le document EP-A-293795. Comme particules de ce type utilisables dans l'invention, on peut citer celles vendues sous les dénominations de Tospearl 103, Tospearl 105 et Tospearl 108 par la société Toshiba.

Ces particules sont connues pour conférer aux compositions qui les contiennent de bonnes propriétés d'étalement et antiseptiques. On peut se référer à ce sujet au document JP-A-05148120. Cependant, l'utilisation de tensioactifs reste dans ce document indispensable. En outre, toutes les particules de polyalkylsilsesquioxane citées dans ce document ne conviennent pas pour stabiliser une émulsion, comme le montrent les exemples comparatifs donnés ci-dessous.

Pour pouvoir stabiliser une émulsion, les particules de polyalkylsilsesquioxane utilisables dans la présente invention doivent avoir un diamètre allant de 7 à 800 nm, et notamment un diamètre inférieur à 14 nm, et par exemple de 7 à 12 nm lorsqu'elles sont obtenues par traitement de surface de silice pyrogénée, et un diamètre de 100 à 800 nm lorsqu'elles sont obtenues par polymérisation d'un alcoxyalkylsilane.

L'émulsion conforme à l'invention comporte par exemple de 1 % à 20 % en poids de particules sphériques par rapport au poids total de l'émulsion, et de préférence de 1 % à 10 % en poids, et mieux de 2 % à 5 % en poids.

La quantité d'huile que l'on peut introduire dans l'émulsion peut représenter de 40 % à 80 % en poids par rapport au poids total de l'émulsion. Elle dépend en pratique de l'origine des particules sphériques.

Quand celles-ci sont obtenues par traitement de surface de silice pyrogénée, la phase huileuse peut aller de 50 % à 80 % en poids par rapport au poids total de l'émulsion, et mieux de 60 % à 75 % en poids.

Quand les particules sont obtenues par polymérisation d'alcoxyalkylsilane, la phase huileuse peut aller de 40 % à 60 % en poids par rapport au poids total de l'émulsion, et mieux de 45 % à 55 % en poids.

De manière générale, si l'on veut incorporer davantage d'huile, on peut ajouter un gélifiant huileux qui permet d'augmenter la quantité d'huile tout en conservant une bonne stabilité de l'émulsion et tout en évitant l'aspect gras lors de l'application de cette émulsion sur la peau. On peut citer comme gélifiant huileux les argiles modifiées comme les bentones, les sels métalliques d'acide gras comme le stéarate d'aluminium, la silice hydrophobe, et les esters de glycol stéarate tels que l'ester acétylé de glycol stéarate vendu sous la dénomination d'Unitwix par la société Guardian.

Comme huiles utilisables dans l'invention, on peut citer les huiles végétales (huile d'abricot), les huiles d'origine animale, les huiles minérales (huile de vaseline), les huiles de synthèse (myristate d'isopropyle, palmitate d'octyle), les huiles siliconées et/ou fluorées. On utilise de préférence des huiles non polaires et/ou de faible poids moléculaire, et notamment les huiles légères telles que l'isohexadécane et les silicones volatiles, par exemple les cyclométhicones. Les huiles légères sont celles présentant une viscosité inférieure ou égale à 0,015 Pa.s. Les huiles non polaires et/ou présentant une viscosité inférieure ou égale à 0,015 Pa.s représentent de préference au moins 50 % en poids de la phase huileuse.

La phase huileuse de l'émulsion peut, en outre, pour améliorer la consistance de l'émulsion, contenir d'autres corps gras tels que les acides gras, les alcools gras et les cires.

Les émulsions objet de l'invention trouvent leur application dans un grand nombre de traitements cosmétiques et/ou dermatologiques de la peau, y compris le cuir chevelu, notamment pour le soin et le maquillage de la peau.

De façon connue, les émulsions de l'invention peuvent contenir des adjuvants habituels dans le domaine cosmétique, tels que des actifs hydrophiles ou lipophiles, des conservateurs, des antioxydants, des parfums, des charges, des filtres, des matières colorantes et encore des vésicules lipidiques. Ces adjuvants, selon leur nature, sont utilisés dans les proportions habituelles des émulsions, et par exemple de 0,01 % à 10 % en poids par rapport au poids total de l'émulsion, et ils sont, selon leur nature, introduits dans la phase aqueuse ou dans la phase huileuse de l'émulsion.

Du fait de la présence possible d'une quantité importante d'huile dans l'émulsion, il est plus facile d'y incorporer des actifs lipophiles. On peut notamment citer comme actifs lipophiles utilisables dans l'invention les vitamines liposolubles telles que le tocophérol (vitamine E) et ses esters, le rétinol (vitamine A) et ses esters, et la vitamine F.

Les exemples ci-après de compositions selon l'invention sont donnés à titre d'illustration et sans caractère limitatif. Les quantités y sont données en % en poids.

### Exemple 1 : Crème

| *Phase huileuse :* | |
|---|---|
| Cyclométhicone | 38,4 % |
| Huile d'abricot | 5,0 % |
| Particules de polyméthylsilsesquioxane (Tospearl 103 vendues par la société Toshiba - diamètre : 300 nm) | 5,0 % |
| Propylparabène (conservateur) | 0,1 % |

| *Phase aqueuse :* | |
|---|---|
| Glycérine | 3,0 % |
| Méthylparabène (conservateur) | 0,2 % |
| Eau | qsp 100,0 % |

Le mode opératoire consiste à disperser, sous agitation magnétique, les particules de polyméthylsilsesquioxane dans la phase huileuse à la température de 25°C, puis à introduire la phase aqueuse dans la phase huileuse ainsi obtenue, sous agitation au Moritz à grande vitesse (3000 tours/minute).

On obtient une émulsion fluide fine, dont les globules d'eau ont un diamètre d'environ 1 µm, utilisable notamment comme crème de jour.

### Exemple comparatif 1 :

| *Phase huileuse :* | |
|---|---|
| Cyclométhicone | 38,4 % |
| Huile d'abricot | 5,0 % |
| Particules de polyméthylsilsesquioxane (Tospearl 240 vendues par la société Toshiba - diamètre : 4000 nm) | 5,0 % |
| Propylparabène (conservateur) | 0,1 % |

| *Phase aqueuse :* | |
|---|---|
| Glycérine | 3,0 % |
| Méthylparabène (conservateur) | 0,2 % |
| Eau | qsp 100,0 % |

Le mode opératoire est le même que dans l'exemple 1.

On obtient une émulsion très grossière dont les globules d'eau ont un diamètre de 10 à 50 µm, dans laquelle il se produit un relargage de l'huile : l'huile se sépare de l'émulsion et remonte à la surface.

### Exemple 2 : Crème

| *Phase huileuse :* | |
|---|---|
| Cyclométhicone | 66,9 % |
| Huile d'abricot | 5,0 % |
| Particules de polyméthylsilsesquioxane (Aerosil R812 vendues par la société Degussa - diamètre : 7 nm) | 5,0 % |
| Propylparabène (conservateur) | 0,1 % |

| *Phase aqueuse :* | |
|---|---|
| Glycérine | 3,0 % |
| Méthylparabène (conservateur) | 0,2 % |
| Eau | qsp 100,0 % |

Le mode opératoire est le même que celui de l'exemple 1. On obtient une émulsion fluide assez fine, dont les globules d'eau ont un diamètre d'environ 800 nm, bien stable.

### Exemple comparatif 2 :

| *Phase huileuse :* | |
|---|---|
| Cyclométhicone | 66,9 % |
| Huile d'abricot | 5,0 % |
| Particules de polyméthylsilsesquioxane (Aerosil R972 vendues par la société Degussa - diamètre : 16 nm) | 5,0 % |
| Propylparabène (conservateur) | 0,1 % |

| *Phase aqueuse :* | |
|---|---|
| Glycérine | 3,0 % |
| Méthylparabène (conservateur) | 0,2 % |
| Eau | qsp 100,0 % |

Le mode opératoire est le même que celui de l'exemple 1. On obtient une émulsion grossière dont les globules d'eau ont un diamètre d'environ 5 à 10 µm, dans laquelle se produit un relargage de l'huile.

### Exemple 3 : Crème

| *Phase huileuse :* | |
|---|---|
| Cyclométhicone | 28,4 % |
| Huile d'abricot | 5,0 % |
| Bentone | 10,0 % |
| Particules de polyméthylsilsesquioxane (Tospearl 103 vendues par la société Toshiba - diamètre : 300 nm)) | 3,5 % |
| Propylparabène (conservateur) | 0,1 % |

| *Phase aqueuse :* | |
|---|---|
| Glycérine | 3,0 % |
| Méthylparabène (conservateur) | 0,2 % |
| Eau | qsp 100,0 % |

Le procédé de préparation consiste à disperser, sous agitation magnétique, les particules de polyméthylsilsesquioxane dans la phase huileuse ne contenant pas la bentone à la température de 25°C, à verser ensuite la phase aqueuse dans la phase huileuse ainsi obtenue sous agitation au Moritz à grande vitesse (3000 tours/minute), puis à ajouter la bentone.

On obtient une émulsion fine, dont les globules d'eau ont un diamètre de 800 nm et qui constitue une crème blanche gélifiée assez épaisse, utilisable notamment comme crème de jour. Elle convient particulièrement bien pour les peaux normales et sèches.

La crème obtenue a été testée par un panel de 31 personnes expertes dans le domaine cosmétique qui l'ont appliquée sur le visage et l'ont gardée toute la journée. La crème a été jugée confortable à l'application, pas collante, peu grasse et douce.

### Obtention des particules de formule A :

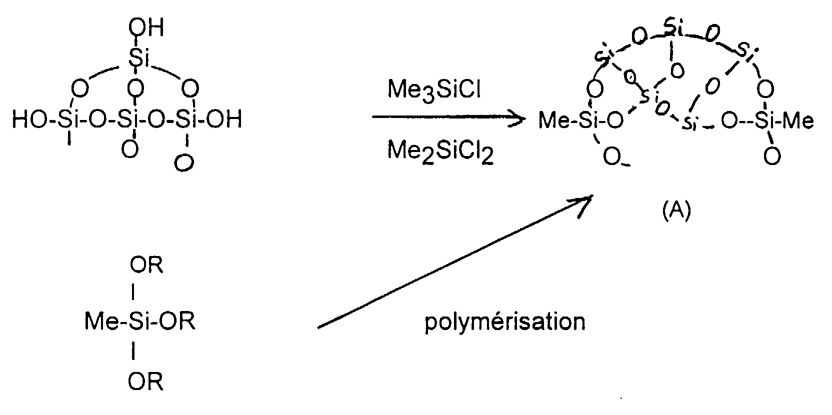

## Revendications

1. Emulsion d'une phase aqueuse dans une phase huileuse, contenant des particules sphériques solides de polyalkylsilsesquioxane, caractérisée en ce qu'elle est exempte de tensioactif, les particules assurant la dispersion de la phase aqueuse dans la phase huileuse, en ce qu'elle présente des globules ayant un diamètre de 0,1 µm à 20 µm et en ce que les particules de polyalkylsilsequioxane sont choisies parmi celles obtenues par un traitement de surface de silice pyrogénée par un alkylsilane éventuellement halogéné et ayant un diamètre inférieur à 14 nm et celles obtenues par polymérisation d'un alcoxyalkylsilane et ayant un diamètre de 100 à 800 nm.

2. Emulsion selon la revendication 1, caractérisée en ce qu'elle est exempte de solvant organique.

3. Emulsion selon la revendication 1 ou 2, caractérisée en ce que l'alkylsilane est un méthylsilane halogéné.

4. Emulsion selon la revendication 1 ou 2, caractérisée en ce que l'alcoxyalkylsilane est un trialcoxyméthylsilane dont le groupement alcoxy comporte de 1 à 5 atomes de carbone.

5. Emulsion selon l'une quelconque desa revendication 1 à 4, caractérisée en ce que les particules représentent de 1 % à 20 % en poids par rapport au poids total de l'émulsion.

6. Emulsion selon l'une quelconque des revendications 1 à 5, caractérisée en ce que les particules représentent de 1 % à 10 % en poids par rapport au poids total de l'émulsion.

7. Emulsion selon l'une quelconque des revendications 1 à 6, caractérisée en ce que la phase huileuse représente de 40 % à 80 % en poids par rapport au poids total de l'émulsion.

8. Emulsion selon l'une quelconque des revendications 1 à 7, caractérisée en ce que la phase huileuse contient une huile non polaire et/ou présentant une viscosité inférieure à 0,015 Pa.s.

9. Emulsion selon la revendication 8, caractérisée en ce que l'huile non polaire et/ou présentant une viscosité inférieure à 0,015 Pa.s est une silicone volatile.

10. Emulsion selon les revendication 8 ou 9, caractérisée en ce que l'huile non polaire et/ou présentant une viscosité inférieure à 0,015 Pa.s représente au moins 50 % en poids de la phase huileuse.

11. Emulsion selon l'une quelconque des revendications 1 à 10, caractérisée en ce qu'elle contient au moins un additif choisi parmi les gélifiants lipophiles, les actifs hydrophiles, les actifs lipophiles, les conservateurs, les antioxydants, les parfums, les charges, les filtres, les matières colorantes et les vésicules lipidiques.

12. Composition cosmétique et/ou dermatologique comprenant une émulsion selon l'une quelconque des revendications 1 à 11.

13. Utilisation de l'émulsion selon l'une quelconque des revendications 1 à 12 pour le traitement cosmétique de la peau.

14. Utilisation de l'émulsion selon l'une quelconque des revendications 1 à 12 pour l'obtention d'une crème destinée au traitement dermatologique de la peau.

15. Procédé de traitement cosmétique caractérisé en ce qu'il consiste à appliquer sur la peau l'émulsion selon l'une quelconque des revendications 1 à 12.

16. Utilisation de particules de polyalkylsilsesquioxane pour disperser une phase aqueuse dans une phase huileuse et stabiliser l'émulsion ainsi obtenue exempte de tensioactif, la dite émulsion présentant des globules dont le diamètre va de 0,1 µm à 20 µm et les particules étant choisies parmi celles obtenues par un traitement de surface de silice pyrogénée par un alkylsilane éventuellement halogéné et ayant un diamètre inférieur à 14 nm et celles obtenues par polymérisation d'un alcoxyalkylsilane et ayant un diamètre de 100 à 800 nm.

## Patentansprüche

1. Emulsion einer wässerigen Phase in einer Ölphase, die feste kugelförmige Polyalkylsilsesquioxan-Partikel enthält, dadurch gekennzeichnet, daß sie keinen grenzflächenaktiven Stoff enthält, wobei die Partikel die Dispersion der wässerigen Phase in der Ölphase gewährleisten, und dadurch, daß sie Kügelchen aufweist, deren Durchmesser im Bereich von 0,1 µm bis 20 µm liegt, sowie dadurch, daß die Polyalkylsilsesquioxan-Partikel unter den Partikeln, die durch Oberflächenbehandlung von pyrogener Kieselsäure mit einem gegebenenfalls halogenierten Alkylsilan hergestellt sind und einen Durchmesser unter 14 nm aufweisen, und unter den Partikeln, die durch Polymerisation eines Alkoxyalkylsilans hergestellt sind und einen Durchmesser von 100 bis 800 nm aufweisen, ausgewählt sind.

2. Emulsion nach Anspruch 1, dadurch gekennzeichnet, daß sie kein organisches Lösemittel enthält.

3. Emulsion nach Anspruch 1 oder 2, dadurch gekennzeichnet, daß das Alkylsilan ein halogeniertes Methylsilan ist.

4. Emulsion nach Anspruch 1 oder 2, dadurch gekennzeichnet, daß das Alkoxyalkylsilan ein Trialkoxymethylsilan ist, dessen Alkoxygruppe 1 bis 5 Kohlenstoffatome aufweist.

5. Emulsion nach einem der Ansprüche 1 bis 4, dadurch gekennzeichnet, daß die Partikel 1 bis 20 Gew.-%, bezogen auf das Gesamtgewicht der Emulsion, ausmachen.

6. Emulsion nach einem der Ansprüche 1 bis 5, dadurch gekennzeichnet, daß die Partikel 1 bis 10 Gew.-%, bezogen auf das Gesamtgewicht der Emulsion, ausmachen.

7. Emulsion nach einem der Ansprüche 1 bis 6, dadurch gekennzeichnet, daß die Ölphase 40 bis 80 Gew.-%, bezogen auf das Gesamtgewicht der Emulsion, ausmacht.

8. Emulsion nach einem der Ansprüche 1 bis 7, dadurch gekennzeichnet, daß die Ölphase ein Öl enthält, das nicht polar ist und/oder eine Viskosität unter 0,015 Pa·s aufweist.

9. Emulsion nach Anspruch 8, dadurch gekennzeichnet, daß das Öl, das nicht polar ist und/oder eine Viskosität unter 0,015 Pa·s aufweist, ein flüchtiges Silicon ist.

10. Emulsion nach den Ansprüchen 8 oder 9, dadurch gekennzeichnet, daß das Öl, das nicht polar ist und/oder eine Viskosität unter 0,015 Pa·s aufweist, mindestens 50 Gew.-% der Ölphase ausmacht.

11. Emulsion nach einem der Ansprüche 1 bis 10, dadurch gekennzeichnet, daß sie mindestens einen Zusatzstoff enthält, der unter lipophilen Gelbildnern, hydrophilen Wirkstoffen, lipophilen Wirkstoffen, Konservierungsmitteln, Antioxidantien, Parfums, Füllstoffen, Filtern, Färbemitteln und Lipidvesikeln ausgewählt ist.

12. Kosmetische und/oder dermatologische Zusammensetzung, die eine Emulsion nach einem der Ansprüche 1 bis 11 enthält.

13. Verwendung der Emulsion nach einem der Ansprüche 1 bis 12 zur kosmetischen Behandlung der Haut.

14. Verwendung der Emulsion nach einem der Ansprüche 1 bis 12 zur Herstellung einer Creme, die zur dermatologischen Behandlung der Haut bestimmt ist.

15. Kosmetisches Behandlungsverfahren, dadurch gekennzeichnet, daß es darin besteht, die Emulsion nach einem der Ansprüche 1 bis 12 auf die Haut aufzutragen.

16. Verwendung von Polyalkylsilsesquioxan-Partikeln zum Dispergieren einer wässerigen Phase in einer Ölphase und zum Stabilisieren der so erhaltenen Emulsion ohne grenzflächenaktiven Stoff, wobei die Emulsion Kügelchen aufweist, deren Durchmesser im Bereich von 0,1 µm bis 20 µm liegt und wobei die Partikel unter den Partikeln, die durch eine Oberflächenbehandlung von pyrogener Kieselsäure mit einem gegebenenfalls halogenierten Alkylsilan hergestellt sind und einen Durchmesser unter 14 nm aufweisen, und unter den Partikeln, die durch Polymerisation eines Alkoxyalkylsilans hergestellt sind und einen Durchmesser von 100 bis 800 nm aufweisen, ausgewählt sind.

## Claims

1. Emulsion of an aqueous phase in an oily phase, containing solid spherical particles of polyalkylsilsesquioxane, characterized in that it is free from surfactant, the particles ensuring the dispersion of the aqueous phase in the oily phase, in that it has globules with a diameter of 0.1 µm to 20 µm and in that the polyalkylsilsesquioxane particles are selected from those obtained by a surface treatment of pyrogenic silica with an optionally halogenated alkylsilane and having a diameter of less than 14 nm and those obtained by polymerization of an alkoxyalkylsilane and having a diameter of 100 to 800 nm.

2. Emulsion according to Claim 1, characterized in that it is free from organic solvent.

3. Emulsion according to Claim 1 or 2, characterized in that the alkylsilane is a halogenated methylsilane.

4. Emulsion according to Claim 1 or 2, characterized in that the alkoxyalkylsilane is a trialkoxymethylsilane in which the alkoxy group contains from 1 to 5 carbon atoms.

5. Emulsion according to any one of Claims 1 to 4, characterized in that the particles represent from 1 % to 20 % by weight relative to the total weight of the emulsion.

6. Emulsion according to any one of Claims 1 to 5, characterized in that the particles represent from 1 % to 10 % by weight relative to the total weight of the emulsion.

7. Emulsion according to any one of Claims 1 to 6, characterized in that the oily phase represents from 40 % to 80 % by weight relative to the total weight of the emulsion.

8. Emulsion according to any one of Claims 1 to 7, characterized in that the oily phase contains an oil which is nonpolar and/or has a viscosity lower than 0.015 Pa s.

9. Emulsion according to Claim 8, characterized in that the oil which is nonpolar and/or has a viscosity lower than 0.015 Pa s is a volatile silicone.

10. Emulsion according to Claim 8 or 9, characterized in that the oil which is nonpolar and/or has a viscosity lower than 0.015 Pa s represents at least 50 % by weight of the oily phase.

11. Emulsion according to any one of Claims 1 to 10, characterized in that it contains at least one additive chosen from lipophilic gelling agents, hydrophilic active substances, lipophilic active substances, stabilizers, antioxidants, perfumes, fillers, screening agents, colorant substances and lipid vesicles.

12. Cosmeti-c and/or dermatological composition comprising an emulsion according to any one of Claims 1 to 11.

13. Use of the emulsion according to any one of Claims 1 to 12 for the cosmetic treatment of skin.

14. Use of the emulsion according to any one of Claims 1 to 12 for obtaining a cream intended for the dermatological treatment of skin.

15. Cosmetic treatment process characterized in that it consists in applying to the skin the emulsion according to any one of Claims 1 to 12.

16. Use of polyalkylsilsesquioxane particles for dispersing an aqueous phase in an oily phase and stabilizing the surfactant-free emulsion thus obtained, the said emulsion having globules whose diameter ranges from 0.1 µm to 20 µm and the particles being chosen from those obtained by a surface treatment of pyrogenic silica with an optionally halogenated alkylsilane and having a diameter of less than 14 nm and those obtained by polymerization of an alkoxyalkylsilane and having a diameter of 100 to 800 nm.
